(19) Europäisches Patentamt
European Patent Office
Office européen des brevets

(11) Numéro de publication : **0 102 283**
**B1**

(12) # FASCICULE DE BREVET EUROPÉEN

(45) Date de publication du fascicule du brevet : **03.12.86**

(51) Int. Cl.[4] : **C 07 D453/02**

(21) Numéro de dépôt : **83401606.5**

(22) Date de dépôt : **02.08.83**

(54) **Nouveau procédé de préparation de dérivés de la quinuclidine substitués en position 3.**

(30) Priorité : **17.08.82 FR 8214215**

(43) Date de publication de la demande : **07.03.84 Bulletin 84/10**

(45) Mention de la délivrance du brevet : **03.12.86 Bulletin 86/49**

(84) Etats contractants désignés : **BE CH DE FR GB IT LI LU NL SE**

(56) Documents cités :
**FR-A- 2 034 605**
**FR-A- 2 052 991**
**COLLECTION OF CZECHOSLOVAK CHEMICAL COMMUNICATIONS, vol. 15, 1950, pages 150-155, Prague, CS R. LUKES et al.: "Sur quelques dérivés bêta-substitués de la quinuclidine"**
**HELVETICA CHIMICA ACTA, vol. 37, no. 196, 1954, pages 1689-1698, Bâle, CH C.A. GROB et al.: "Untersuchungen in der Chinuclidin-Reihe"**

(73) Titulaire : **PHARMUKA LABORATOIRES**
**35 Quai du Moulin de Cage**
**F-92231 Gennevilliers (FR)**

(72) Inventeur : **Bondiou, Jean-Claude**
**36, Place Jules Ferry**
**F-92120 Montrouge (FR)**
Inventeur : **Hodac, Françoise**
**5 bis, rue Dosne**
**F-75116 Paris (FR)**
Inventeur : **Legroux, Didier Alain Michel**
**70 Domont-Village**
**F-95330 Domont (FR)**

(74) Mandataire : **Gaumont, Robert et al**
**RHONE-POULENC RECHERCHES Service Brevets**
**Pharma 25, Quai Paul Doumer**
**F-92408 Courbevoie Cedex (FR)**

**EP 0 102 283 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet un nouveau procédé de préparation des dérivés de la quinuclidine de formule :

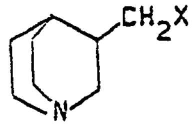

(I)

dans laquelle X est un atome de chlore, de brome ou d'iode ou un groupe hydroxy.

Les composés de formule (I) sont des produits connus, utilisables en particulier comme produits intermédiaires pour la préparation de médicaments (cf. par exemple les brevets français n° 2 034 605 et 2 052 991). Ils ont été préparés à partir de la quinuclidinone-3 par C. A. GROB et E. RENK (Helvetica Chimica Acta 1954, Vol. 37, p. 1689-1698) selon un procédé comportant au minimum 6 étapes, qui peut être schématisé comme suit :

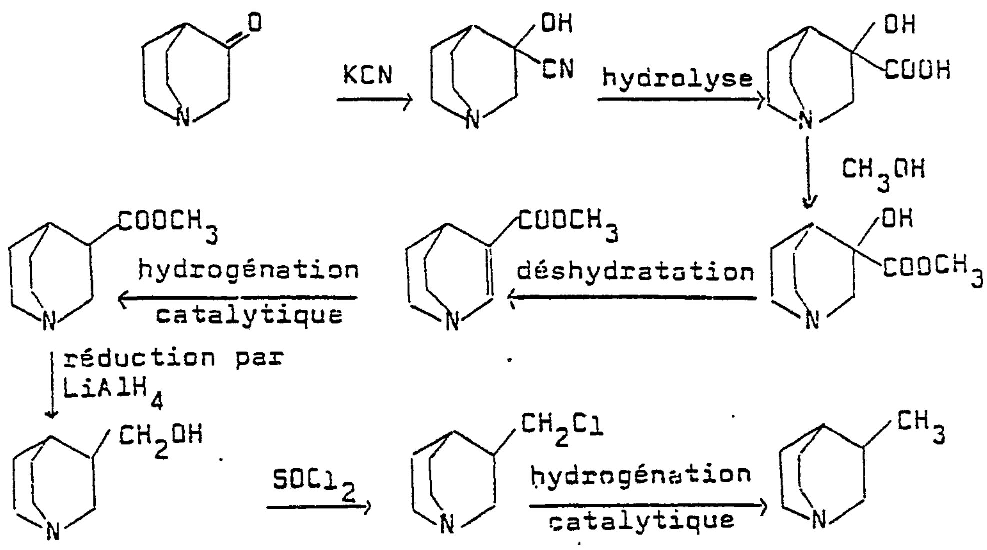


Le procédé selon l'invention consiste :

a) à faire réagir, au sein d'un solvant approprié et en présence d'une quantité catalytique d'un halogénure de métal de transition, la méthylène-3 quinuclidine de formule :

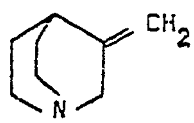

(II)

avec un hydrure d'aluminium, le rapport molaire

$$\frac{\text{hydrure d'aluminium}}{\text{méthylène-3 quinuclidine}}$$

étant au moins égal à 0,6,

b) à faire réagir *in situ* sur le produit formé dans l'étape a) un ester,

c) à faire réagir *in situ* sur le produit formé dans l'étape b) un réactif électrophile susceptible de céder un atome ou groupe X, X ayant la signification indiquée dans la formule (I).

Comme solvant utilisable dans l'étape a) on peut citer par exemple le tétrahydrofuranne, le diméthoxyéthane (ou éther diméthylique de l'éthylèneglycol), le diglyme, le triglyme, les mélanges entre eux des composés précédents, et les mélanges de ces composés avec un hydrocarbure aromatique tel que le benzène ou le toluène contenant au plus 50 % en volume d'hydrocarbure aromatique. Comme halogénure de métal de transition utilisable dans l'étape a) on peut citer en particulier le tétrachlorure de titane, le tétrachlorure de zirconium, le chlorure de cobalt et le chlorure de nickel. Comme hydrure d'aluminium utilisable dans l'étape a) on peut citer en particulier l'hydrure de lithium et d'aluminium $LiAlH_4$ et l'hydrure de bis (méthoxy-2 éthoxy) aluminium. L'étape a) est effectuée à une température comprise entre la température ambiante (15 °C à 25 °C) et la température d'ébullition du solvant. Elle est

effectuée de préférence à la température ambiante.

Dans le cas où on emploie dans l'étape a) $LiAlH_4$ comme hydrure d'aluminium et $TiCl_4$ comme halogénure de métal de transition, on opère de préférence avec un rapport molaire $LiAlH_4$/méthylène-3 quinuclidine de 1,2 à 1,3 et un rapport molaire $LiAlH_4/TiCl_4$ compris entre 10 et 25, et plus particulièrement voisin de 17. Dans ces conditions, et en opérant à la température ambiante au sein d'un mélange toluène-tétrahydrofuranne (ou diméthoxyétane) contenant 3 parties en volume de toluène pour 5 parties en volume de tétrahydrofuranne (ou de diméthoxyéthane), l'étape a) est terminée au bout de 24 heures d'agitation du milieu réactionnel.

Comme esters utilisables dans l'étape b) on peut citer en particulier les esters des alcools aliphatiques saturés ayant 1 à 6 atomes de carbone avec les acides monocarboxyliques aliphatiques saturés ayant 1 à 6 atomes de carbone, et plus spécialement l'acétate d'éthyle et le formiate de méthyle. L'étape b) est effectuée de préférence à une température comprise entre — 5 °C et la température ambiante.

Comme réactifs électrophiles utilisables dans l'étape c), on peut citer :

— en tant que réactifs susceptibles de céder un atome de chlore, le chlore, le chlorure cuivrique et le N-chloro succinimide,

— en tant que réactifs susceptibles de céder un atome de brome, le brome, le bromure cuivrique, le N-bromo succinimide et la dibromohydantoïne,

— en tant que réactif susceptible de céder un atome d'iode,

— en tant que réactifs susceptibles de céder un groupe hydroxy, l'eau oxygénée et les hydroperoxydes organiques (par exemple l'hydroperoxyde de tertiobutyle).

Les conditions de température utilisées dans l'étape c) sont fonction du réactif électrophile mis en jeu. Le tableau ci-dessous donne des conditions de température utilisables pour certains des réactifs électrophiles cités précédemment.

| Réactif électrophile utilisé dans l'étape c) | Température utilisable dans l'étape c) |
|---|---|
| Chlore | 0°C à - 5°C |
| Brome | - 5°C à la température ambiante |
| Iode | - 5°C à la température ambiante |
| Eau oxygénée | - 50°C à - 60°C |
| Hydroperoxyde de tertiobutyle | - 60°C à la température ambiante |
| Chlorure cuivrique | - 5°C à la température ambiante |

La méthylène-3 quinuclidine utilisée comme produit de départ dans le procédé selon l'invention est un composé connu, qui peut être préparé en particulier par action du bromure de triphénylméthylphosphonium sur la quinuclidinone-3 (réaction de WITTIG). Le procédé selon l'invention permet donc d'obtenir les composés de formule (I) à partir de la quinuclidinone-3 en seulement quatre étapes et sans qu'il soit nécessaire d'isoler les produits intermédiairement formés.

Les exemples suivants illustrent l'invention sans la limiter.

Exemple 1 : Préparation de la chlorométhyl-3 quinuclidine

a) A une suspension de 3,8 g (0,1 mole) d'hydrure de lithium et d'aluminium dans 50 $cm^3$ de tétrahydrofuranne, placée sous atmosphère d'azote et maintenue à la température de 20 °C, on ajoute, sous agitation, une solution de 9,8 g (0,08 mole) de méthylène-3 quinuclidine dans 30 $cm^3$ de toluène. Après 15 minutes d'agitation du milieu réactionnel, on ajoute 0,6 $cm^3$ de tétrachlorure de titane en

maintenant la température vers 15 °C. La suspension noire obtenue est ensuite agitée pendant 24 heures à la température ambiante. Au bout de ce laps de temps, le milieu réactionnel ne contient plus de méthylène-3 quinuclidine ainsi que le montre un contrôle effectué par chromatographie en phase gazeuse.

b) La suspension obtenue à la fin de l'étape a) ci-dessus est refroidie à — 5 °C. On ajoute alors goutte à goutte, en maintenant la température inférieure ou égale à 5 °C, 17 g (0,19 mole) d'acétate d'éthyle anhydre et agite la suspension pendant une heure à la température ambiante.

c) La suspension obtenue à la fin de l'étape b) ci-dessus est refroidie à — 5 °C, puis on fait barboter doucement dans le milieu réactionnel un courant de chlore, la température du milieu étant maintenue inférieure ou égale à 0 °C. Le milieu réactionnel se décolore progressivement. On arrête le barbotage de chlore lorsque 23 g (0,32 mole) de chlore ont été absorbés.

On maintient ensuite le milieu réactionnel sous agitation pendant une heure à 0 °C, puis on ajoute lentement, sans dépasser la température de 5 °C, 100 $cm^3$ d'une solution aqueuse N d'acide chlorhydrique. Après agitation, la phase aqueuse est décantée, lavée par deux fois 40 $cm^3$ de toluène, puis refroidie à 0 °C et alcalinisée par addition de 150 $cm^3$ d'une solution aqueuse à 30 % d'hydroxyde de sodium. Le produit formé est extrait par trois fois 30 $cm^3$ de toluène. Les phases toluéniques rassemblées sont lavées avec 15 $cm^3$ d'eau, puis séchées sur sulfate de sodium et on ajoute 30 $cm^3$ d'une solution (environ 5N) d'acide chlorhydrique dans l'isopropanol. Les cristaux obtenus sont filtrés et lavés à l'acétone. On obtient ainsi 11,7 g (ce qui correspond à un rendement de 75 % par rapport à la méthylène-3 quinuclidine mise en jeu) de chlorhydrate de chlorométhyl-3 quinuclidine, qui se sublime à 265 °C.

Exemple 2 : Préparation de la bromométhyl-3 quinuclidine

a) On opère comme dans l'étape a) de l'exemple 1 en partant de 5,7 g d'hydrure de lithium et d'aluminium dans 75 $cm^3$ de tétrahydrofuranne, 14,7 g (0,119 mole) de méthylène-3 quinuclidine dans 45 $cm^3$ de toluène, et 0,9 $cm^3$ de tétrachlorure de titane.

b) A la suspension obtenue à la fin de l'étape a), on ajoute, en maintenant la température du milieu vers 0 °C, 30 ml d'acétate d'éthyle anhydre. Le milieu réactionnel est ensuite agité pendant une heure à la température ambiante.

c) Le milieu réactionnel obtenu à la fin de l'étape b) ci-dessus est refroidi à — 5 °C, puis on ajoute 22,2 g (0,139 mole) de brome, la température du milieu étant maintenue inférieure ou égale à + 5 °C. On laisse ensuite le milieu réactionnel sous agitation pendant 4 heures à la température ambiante.

On ajoute ensuite successivement, en maintenant la température du milieu entre 10 et 15 °C, 5,7 ml d'eau, 4,5 ml d'une solution aqueuse à 20 % d'hydroxyde de sodium et 19,8 ml d'eau. Le précipité obtenu est filtré et lavé avec du toluène. Le filtrat et le toluène ayant servi au lavage du précipité sont rassemblés et on ajoute une solution d'acide chlorhydrique dans l'éthanol jusqu'à ce que le pH du milieu atteigne la valeur 1. On concentre alors le milieu par distillation sous vide et reprend le résidu avec 100 ml d'acétone. Les cristaux obtenus sont séparés par filtration. On obtient ainsi 19 g (ce qui correspond à un rendement de 66,6 % par rapport à la méthylène-3 quinuclidine mise en jeu) de chlorhydrate de bromométhyl-3 quinuclidine, qui fond à 264-266 °C.

Exemple 3 : Préparation de l'iodométhyl-3 quinuclidine

a) On opère comme dans l'étape a) de l'exemple 2.

b) On opère comme dans l'étape b) de l'exemple 2.

c) Le milieu réactionnel obtenu à la fin de l'étape b) est refroidi à — 5 °C, puis on ajoute 35,5 g (0,14 mole) d'iode en solution dans 300 ml de toluène, la température du milieu étant maintenue entre 0 °C et + 5 °C. On laisse ensuite le milieu réactionnel sous agitation pendant 4 heures à la température ambiante.

Le milieu réactionnel est ensuite traité comme indiqué à la partie c) de l'exemple 2. On obtient ainsi 17 g (ce qui correspond à un rendement de 50 % par rapport à la méthylène-3 quinuclidine mise en jeu) de chlorhydrate d'iodométhyl-3 quinuclidine, qui fond à 196-198 °C en se décomposant.

Exemple 4 : Préparation de l'hydroxyméthyl-3 quinuclidine

a) On opère comme dans l'étape a) de l'exemple 1 en partant de 4,8 g d'hydrure de lithium et d'aluminium dans 60 $cm^3$ de tétrahydrofuranne, 12,3 g (0,1 mole) de méthylène-3 quinuclidine dans 40 $cm^3$ de toluène, et 0,75 $cm^3$ de tétrachlorure de titane.

b) On opère comme dans l'étape b) de l'exemple 2.

c) Le milieu réactionnel obtenu à la fin de l'étape b) est refroidi à — 60 °C et placé sous courant d'azote, puis on ajoute goutte à goutte 13 g d'une solution aqueuse à 30 % d'eau oxygénée (soit 0,115 mole d'eau oxygénée), la température du milieu étant maintenue inférieure ou égale à — 50 °C. Le milieu réactionnel est ensuite maintenu à — 60 °C pendant une heure.

On ajoute ensuite au milieu 80 $cm^3$ de lessive de soude, laisse la température remonter vers 20 °C et extrait par quatre fois 80 $cm^3$ d'oxyde diéthylique. Les phases oxyde diéthylique sont rassemblées,

séchées et le solvant est éliminé par distillation. Le résidu obtenu est repris avec 80 $cm^3$ d'éther de pétrole. Les cristaux obtenus sont séparés par filtration et séchés sous vide en présence de $P_2O_5$. On obtient ainsi 4,5 g (ce qui correspond à un rendement de 32,6 % par rapport à la méthylène-3 quinuclidine mise en jeu) d'hydroxyméthyl-3 quinuclidine, qui fond à 59 °C.

Exemple 5 : Préparation de l'hydroxyméthyl-3 quinuclidine

a) On opère comme dans l'étape a) de l'exemple 1 en partant de 48 g d'hydrure de lithium et d'aluminium dans 600 $cm^3$ de tétrahydrofuranne, 123 g (1 mole) de méthylène-3 quinuclidine dans 400 $cm^3$ de toluène, et 7,5 $cm^3$ de tétrachlorure de titane.

b) A la suspension obtenue à la fin de l'étape a), on ajoute, en maintenant la température du milieu vers 0 °C, 300 ml d'acétate d'éthyle anhydre. Après la fin de l'introduction, le milieu réactionnel est agité pendant une heure à la température ambiante.

c) Le milieu obtenu à la fin de l'étape b) est refroidi vers — 60 °C et on ajoute lentement 95 g (1,05 mole) d'hydroperoxyde de tertiobutyle. Le milieu réactionnel est ensuite agité pendant 17 heures à la température ambiante.

On ajoute ensuite successivement au milieu, maintenu à une température de 0 °C à + 10 °C et placé sous atmosphère d'azote, 32 ml d'eau, puis 67 ml d'une solution aqueuse à 30 % d'hydroxyde de sodium et enfin 85 ml d'eau. Le précipité obtenu est filtré, lavé avec du toluène. Le filtrat et le toluène ayant servi au lavage du précipité sont rassemblés et concentrés par distillation sous pression réduite. Le résidu est repris avec 300 ml d'éther diisopropylique. Les cristaux obtenus sont séparés par filtration. On obtient ainsi 63 g (ce qui correspond à un rendement de 45 % par rapport à la méthylène-3 quinuclidine mise en jeu) d'hydroxyméthyl-3 quinuclidine.

Exemple 6 : Préparation de la chlorométhyl-3 quinuclidine

a) On opère comme dans l'étape a) de l'exemple 1 en partant de 4,7 g d'hydrure d'aluminium et de lithium dans 60 ml de tétrahydrofuranne, 12,3 g de méthylène-3 quinuclidine (0,1 mole) dans 40 $cm^3$ de toluène et 0,7 ml de tétrachlorure de titane.

b) A la suspension obtenue à la fin de l'étape a), on ajoute, en maintenant la température du milieu vers 0 °C, 25 ml d'acétate d'éthyle anhydre, et on agite la suspension pendant une heure à la température ambiante.

c) Le milieu obtenu à la fin de l'étape b) est refroidi à une température de 0 °C à — 5 °C, et on ajoute lentement 27 g (0,2 mole) de chlorure cuivrique, la température du milieu étant maintenue inférieure ou égale à + 5 °C pendant l'addition.

On maintient ensuite le milieu réactionnel sous agitation pendant une heure à la température ambiante. On ajoute alors, en refroidissant vers 10 °C, 15 ml d'eau et 25 ml d'une solution aqueuse concentrée à 37 % d'acide chlorhydrique. Le précipité de chlorure cuivreux formé est séparé par filtration et lavé avec 10 ml d'eau. La phase organique est éliminée par décantation et les phases aqueuses rassemblées sont alcalinisées par addition d'une solution aqueuse à 32 % d'hydroxyde de sodium jusqu'à ce que le pH du milieu aqueux soit environ 14, la température du milieu étant maintenue entre 10 °C et 20 °C pendant l'alcalinisation.

Le milieu aqueux est alors extrait avec 3 fois 40 ml de toluène. Les phases toluéniques rassemblées sont lavées avec 20 ml d'eau, puis séchées sur sulfate de sodium et on ajoute 40 $cm^3$ d'une solution 5N d'acide chlorhydrique dans l'isopropanol. Les cristaux formés sont filtrés et lavés à l'acétone. On obtient ainsi 12,5 g (ce qui correspond à un rendement de 64 % par rapport à la méthylène-3 quinuclidine mise en jeu) de chlorhydrate de chlorométhyl-3 quinuclidine.

Exemple 7 : Préparation de la chlorométhyl-3 quinuclidine :

a) On opère comme dans l'étape a) de l'exemple 1, en partant de 2,28 g d'hydrure d'aluminium et de lithium dans 50 ml de tétrahydrofuranne, 6,15 g de méthylène-3 quinuclidine (0,05 mole) et 0,7 g de tétrachlorure de zirconium (à la place de 0,6 $cm^3$ de tétrachlorure de titane).

b) On opère comme dans l'étape b) de l'exemple 6.

c) On opère comme dans l'étape c) de l'exemple 6, en utilisant 13,5 g (0,1 mole) de chlorure cuivrique au lieu de 27 g de chlorure cuivrique.

On termine l'opération comme indiqué à l'exemple 6.

On obtient ainsi 4,68 g de chlorhydrate de chlorométhyl-3 quinuclidine (ce qui correspond à un rendement de 48 % par rapport à la méthylène-3 quinuclidine mise en jeu).

**Revendications**

1. Procédé de préparation des composés de formule :

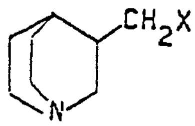

(I)

dans laquelle X est un atome de chlore, de brome ou d'iode ou un groupe hydroxy, caractérisé en ce que :

a) on fait réagir, au sein d'un solvant approprié et en présence d'une quantité catalytique d'un halogénure de métal de transition, la méthylène-3 quinuclidine de formule :

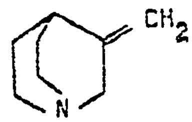

(II)

avec un hydrure d'aluminium, le rapport molaire

$$\frac{\text{hydrure d'aluminium}}{\text{méthylène-3 quinuclidine}}$$

étant au moins égal à 0,6,

b) on fait réagir *in situ* sur le produit formé dans l'étape a) un ester,

c) on fait réagir *in situ* sur le produit formé dans l'étape b) un réactif électrophile susceptible de céder un atome ou groupe X, X ayant la signification indiquée dans la formule (I).

2. Procédé selon la revendication 1, caractérisé en ce qu'on emploie comme solvant dans l'étape a) le tétrahydrofuranne, le diméthoxyéthane, le diglyme, le triglyme, les mélanges entre eux de ces composés, ou les mélanges de ces composés avec un hydrocarbure aromatique contenant au plus 50 % en volume d'hydrocarbure aromatique.

3. Procédé selon l'une quelconque des revendications 1 et 2, caractérisé en ce que, dans l'étape a), on utilise le tétrachlorure de titane, le tétrachlorure de zirconium, le chlorure de cobalt ou le chlorure de nickel comme halogénure de métal de transition, et l'hydrure de lithium et d'aluminium ou l'hydrure de bis (méthoxy-2 éthoxy) aluminium comme hydrure d'aluminium.

4. Procédé selon la revendication 3, caractérisé en ce que, dans l'étape a), on utilise l'hydrure de lithium et d'aluminium comme hydrure d'aluminium et le tétrachlorure de titane comme halogénure de métal de transition et opère avec un rapport molaire $LiAlH_4$/méthylène-3 quinuclidine de 1,2 à 1,3 et un rapport molaire $LiAlH_4/TiCl_4$ compris entre 10 et 25.

5. Procédé selon l'une quelconque des revendications 1 à 4, caractérisé en ce qu'on utilise comme ester dans l'étape b) un ester d'un alcool aliphatique saturé ayant 1 à 6 atomes de carbone avec un acide monocarboxylique aliphatique saturé ayant 1 à 6 atomes de carbone.

6. Procédé selon la revendication 5, caractérisé en ce qu'on utilise comme ester dans l'étape b) l'acétate d'éthyle ou le formiate de méthyle.

7. Procédé selon l'une quelconque des revendications 1 à 6, caractérisé en ce que le réactif électrophile mis en jeu dans l'étape c) est choisi parmi le chlore, le chlorure cuivrique, le N-chloro succinimide, le brome, le bromure cuivrique, le N-bromo succinimide, la dibromohydantoïne, l'iode, l'eau oxygénée et les hydroperoxydes organiques.

8. Procédé selon la revendication 7 pour la préparation de la chlorométhyl-3 quinuclidine, caractérisé en ce que le réactif électrophile mis en jeu dans l'étape c) est le chlore et l'étape c) est effectuée à une température située dans la zone de 0 °C à — 5 °C.

9. Procédé selon la revendication 7 pour la préparation de la bromométhyl-3 quinuclidine, caractérisé en ce que le réactif électrophile mis en jeu dans l'étape c) est le brome et l'étape c) est effectuée à une température située dans la zone de — 5 °C à la température ambiante.

10. Procédé selon la revendication 7 pour la préparation de l'iodométhyl-3 quinuclidine, caractérisé en ce que le réactif électrophile mis en jeu dans l'étape c) est l'iode et l'étape c) est effectuée à une température située dans la zone de — 5 °C à la température ambiante.

11. Procédé selon la revendication 7 pour la préparation de l'hydroxyméthyl-3 quinuclidine, caractérisé en ce que le réactif électrophile mis en jeu dans l'étape c) est l'eau oxygénée et l'étape c) est effectuée à une température située dans la zone de — 60 °C à — 50 °C.

12. Procédé selon la revendication 7 pour la préparation de l'hydroxyméthyl-3 quinuclidine, caractérisé en ce que le réactif électrophile mis en jeu dans l'étape c) est l'hydroperoxyde de tertiobutyle et l'étape c) est effectuée à une température située dans la zone de — 60 °C à la température ambiante.

13. Procédé selon la revendication 7 pour la préparation de la chlorométhyl-3 quinuclidine, caractérisé en ce que le réactif électrophile mis en jeu dans l'étape c) est le chlorure cuivrique et l'étape c) est effectuée à une température située dans la zone de — 5 °C à la température ambiante.

**Claims**

1. Process for the preparation of compounds of formula :

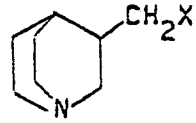

(I)

in which X is a chlorine, bromine or iodine atom or a hydroxy group, characterized in that :

a) 3-methylenequinuclidine of formula :

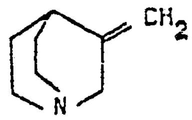

(II)

is reacted, in a suitable solvent and in the presence of a catalytic quantity of a transition metal halide, with an aluminium hydride, the mole ratio

$$\frac{\text{aluminium hydride}}{\text{3-methylenequinuclidine}}$$

being at least equal to 0.6,

b) an ester is reacted *in situ* with the product formed in step a),

c) an electrophilic reactant capable of donating an atom or group X, X having the meaning shown in formula (I) is reacted *in situ* with the product formed in step b).

2. Process according to Claim 1, characterized in that tetrahydrofuran, dimethoxyethane, diglyme, triglyme, mixtures of these compounds with each other, or mixtures of these compounds with an aromatic hydrocarbon containing at most 50 % by volume of aromatic hydrocarbon, are used as a solvent in step a).

3. Process according to either of Claims 1 and 2, characterized in that titanium tetrachloride, zirconium tetrachloride, cobalt chloride or nickel chloride is used as a transition metal halide, and lithium aluminium hydride or bis(2-methoxyethoxy) aluminium hydride is used as an aluminium hydride in step a).

4. Process according to Claim 3, characterized in that, in step a), lithium aluminium hydride is used as the aluminium hydride and titanium tetrachloride is used as the transition metal halide, and the operation is carried out with a $LiAlH_4$/3-methylenequinuclidine mole ratio of 1.2 to 1.3 and a $LiAlH_4/TiCl_4$ mole ratio of between 10 and 25.

5. Process according to any one of Claims 1 to 4, characterized in that an ester of a saturated aliphatic alcohol containing 1 to 6 carbon atoms with a saturated aliphatic monocarboxylic acid containing 1 to 6 carbon atoms is used as the ester in step b).

6. Process according to Claim 5, characterized in that ethyl acetate or methylformate is used as the ester in step b).

7. Process according to any one of Claims 1 to 6, characterized in that the electrophilic reactant used in step c) is chosen from chlorine, cupric chloride, N-chlorosuccinimide, bromine, cupric bromide, N-bromosuccinimide, dibromohydantoin, iodine, hydrogen peroxide and organic hydroperoxides.

8. Process according to Claim 7 for the preparation of 3-chloromethylquinuclidine, characterized in that the electrophilic reactant used in step c) is chlorine and step c) is carried out at a temperature situated in the region from 0 °C to — 5 °C.

9. Process according to Claim 7 for the preparation of 3-bromomethylquinuclidine, characterized in that the electrophilic reactant used in step c) is bromine and step c) is carried out at a temperature situated in the region from — 5 °C to ambient temperature.

10. Process according to Claim 7 for the preparation of 3-iodomethylquinuclidine, characterized in that the electrophilic reactant used in step c) is iodine and step c) is carried out at a temperature situated in the region from — 5 °C to ambient temperature.

11. Process according to Claim 7 for the preparation of 3-hydroxymethylquinuclidine, characterized in that the electrophilic reactant used in step c) is hydrogen peroxide and step c) is carried out at a temperature situated in the region from — 60 °C to — 50 °C.

12. Process according to Claim 7 for the preparation of 3-hydroxymethylquinuclidine, characterized in that the electrophilic reactant used in step c) is tert-butyl hydroperoxide and step c) is carried out at a temperature situated in the region from — 60 °C to ambient temperature.

13. Process according to Claim 7 for the preparation of 3-chloromethylquinuclidine, characterized in that the electrophilic reactant used in step c) is cupric chloride and step c) is carried out at a temperature situated in the region from — 5 °C to ambient temperature.

**Patentansprüche**

1. Verfahren zur Herstellung von Verbindungen der Formel

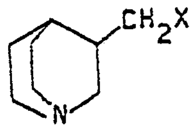

(I)

worin X ein Chlor-, Brom- oder Jodatom oder eine Hydroxygruppe ist, dadurch gekennzeichnet, daß man

a) in einem geeigneten Lösungsmittel und in Gegenwart einer katalytischen Menge eines Übergangsmetallhalogenids 3-Methylenchinuclidin der Formel

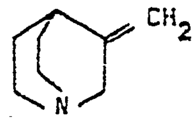

(II)

mit einem Aluminiumhydrid reagieren läßt, wobei das Molverhältnis

$$\frac{\text{Aluminiumhydrid}}{\text{3-Methylenchinuclidin}}$$

wenigstens gleich 0,6 ist,

b) auf das in Stufe a) gebildete Produkt *in situ* einen Ester reagieren läßt,

c) auf das in Stufe b) gebildete Produkt *in situ* ein elektrophiles Reagens reagieren läßt, das fähig ist, ein Atom oder eine Gruppe X abzugeben, wobei X die in Formel (I) angegebene Bedeutung hat.

2. Verfahren gemäß Anspruch 1, dadurch gekennzeichnet, daß man als Lösungsmittel in Stufe a) Tetrahydrofuran, Dimethoxyethan, Diglyme, Triglyme, Mischungen dieser Verbindungen untereinander oder Mischungen dieser Verbindungen mit einem aromatischen Kohlenwasserstoff, die höchstens 50 Vol-% aromatischen Kohlenwasserstoff enthalten, verwendet.

3. Verfahren gemäß einem der Ansprüche 1 und 2, dadurch gekennzeichnet, daß man in Stufe a) Titantetrachlorid, Zirkontetrachlorid, Kobaltchlorid oder Nickelchlorid als Übergangsmetallhalogenid verwendet und Lithium-aluminiumhydrid oder Bis-(2-methoxyethoxy)-aluminiumhydrid als Aluminiumhydrid verwendet.

4. Verfahren gemäß Anspruch 3, dadurch gekennzeichnet, daß man in Stufe a) Lithium-aluminiumhydrid als Aluminiumhydrid verwendet und Titantetrachlorid als Übergangsmetallhalogenid verwendet und mit einem Molverhältnis $LiAlH_4$/3-Methylenchinuclidin von 1,2 bis 1,3 und einem Molverhältnis $LiAlH_4/TiCl_4$ zwischen 10 und 25 arbeitet.

5. Verfahren gemäß einem der Ansprüche 1 bis 4, dadurch gekennzeichnet, daß man als Ester in Stufe b) einen Ester eines gesättigten, aliphatischen Alkohols mit 1 bis 6 Kohlenstoffatomen mit einer gesättigten, aliphatischen Monocarbonsäure mit 1 bis 6 Kohlenstoffatomen verwendet.

6. Verfahren gemäß Anspruch 5, dadurch gekennzeichnet, daß man als Ester in Stufe b) Ethylacetat oder Ameisensäuremethylester verwendet.

7. Verfahren gemäß einem der Ansprüche 1 bis 6, dadurch gekennzeichnet, daß das in Stufe c) eingesetzte elektrophile Reagens ausgewählt ist unter Chlor, Cuprichlorid, N-Chlorsuccinimid, Brom, Cupribromid, N-Bromsuccinimid, Dibromhydantoin, Jod, Wasserstoffperoxid und organischen Hydroperoxiden.

8. Verfahren gemäß Anspruch 7 zur Herstellung von 3-Chlormethylchinuclidin, dadurch gekennzeichnet, daß das in Stufe c) eingesetzte, elektrophile Reagens Chlor ist und die Stufe c) bei einer Temperatur im Bereich von 0 bis —5 °C durchgeführt wird.

9. Verfahren gemäß Anspruch 7 zur Herstellung von 3-Brommethylchinuclidin, dadurch gekennzeichnet, daß das in Stufe c) eingesetzte, elektrophile Reagens Brom ist und die Stufe c) bei einer Temperatur im Bereich von —5 °C bis Umgebungstemperatur durchgeführt wird.

10. Verfahren gemäß Anspruch 7 zur Herstellung von 3-Jodmethylchinuclidin, dadurch gekennzeichnet, daß das in Stufe c) eingesetzte, elektrophile Reagens Jod ist und die Stufe c) bei einer Temperatur im Bereich von —5 °C bis Umgebungstemperatur durchgeführt wird.

11. Verfahren gemäß Anspruch 7 zur Herstellung von 3-Hydroxymethylchinuclidin, dadurch gekennzeichnet, daß das in Stufe c) eingesetzte, elektrophile Reagens Wasserstoffperoxid ist und die Stufe c) bei einer Temperatur im Bereich von —60 bis —50 °C durchgeführt wird.

12. Verfahren gemäß Anspruch 7 zur Herstellung von 3-Hydroxymethylchinuclidin, dadurch gekennzeichnet, daß das in Stufe c) eingesetzte, elektrophile Reagens tert.-Butylhydroperoxid ist und die Stufe c) bei einer Temperatur im Bereich von —60 °C bis Umgebungstemperatur durchgeführt wird.

13. Verfahren gemäß Anspruch 7 zur Herstellung von 3-Chlormethylchinuclidin, dadurch gekennzeichnet, daß das in Stufe c) eingesetzte, elektrophile Reagens Cuprichlorid ist und die Stufe c) bei einer Temperatur im Bereich von —5 °C bis Umgebungstemperatur durchgeführt wird.